# EUROPEAN PATENT APPLICATION

(11) **EP 4 197 495 A1**
(43) Date of publication of application: **21.06.2023**
(21) Application number: 21858479.5
(22) Date of filing: 21.07.2021
(51) Int. Cl.: A61F 2/30

(54) **REINFORCEMENT IMPLANT**

(30) Priority: 21.08.2020 KR 20200105129
(71) Applicant: Corentec Co., Ltd., Seoul 06649 (KR)
(72) Inventor: KIM, Jung-Sung, Seoul 05555 (KR); KANG, Yeo-Kyung, Seongnam-si Gyeonggi-do 13162 (KR); KIM, Seong-Jin, Seoul 06640 (KR)
(74) Representative: Witte, Weller & Partner Patentanwälte mbB
(86) International application number: PCT/KR2021/009437
(87) International publication number: WO 2022/039392

(57) **Abstract**

Proposed is an augment implant and, more specifically, is an augment implant, in which a body part of the porous structure is combined with a frame part of a non-porous structure to increase the mechanical strength of the body part and to prevent damage to the body part due to external forces, bone ingrowth is maximized by minimizing the area of the frame part, the augment implant can be inserted in various directions, not just in a specific direction, and when the augment implant is manufactured by 3D printing, a support is connected to the frame part so that in a process of removing the support, the support can be easily removed without damage to the porous structure.

## Description

### Technical Field

The present disclosure relates to an augment implant and, more specifically, to an augment implant, in which a body part of a porous structure is combined with a frame part of a non-porous structure to increase the mechanical strength of the body part and to prevent damage to the body part due to external forces, bone ingrowth is maximized by minimizing the area of the frame part, the augment implant can be inserted in various directions, not just in a specific direction, and when the augment implant is manufactured by 3D printing, a support is connected to the frame part so that in a process of removing the support, the support can be easily removed without damage to the porous structure.

### Background Art

A joint is a part of a body where two or more bones are in contact with each other as a movable structure. A human joint may have various problems due to damage by repetitive use, arthritis, and aging, etc. When a joint is damaged enough to lose its original function, artificial joint replacement may be performed to replace the damaged joint with an implant.

Artificial joint replacement is performed in a process of manufacturing an implant having a shape complementary to the anatomical shape of a damaged joint, cutting the bone of the damaged joint within a predetermined range, and implanting the implant in the cut area.

In this case, when bone cutting is required entirely or partially, such as when the damaged joint area is extensive, has severe local damage, or requires revision surgery, an augment implant may be used to compensate for a bone area lost by cutting.

The augment implant is a component which supplements a lost bone, and is formed to have a shape complementary to the shape of the lost bone area, so that the augment implant is seated in empty space defined by the cut bone.

In general, such an augment implant may be made of biomaterials (biometals, bioceramics, biopolymers) in a solid form.

However, when the augment implant is configured in a solid form without a pore, a bone cannot grow in an area equal to space occupied by the augment implant.

For this reason, a method of forming the augment implant with a porous material having multiple pores has been sought.

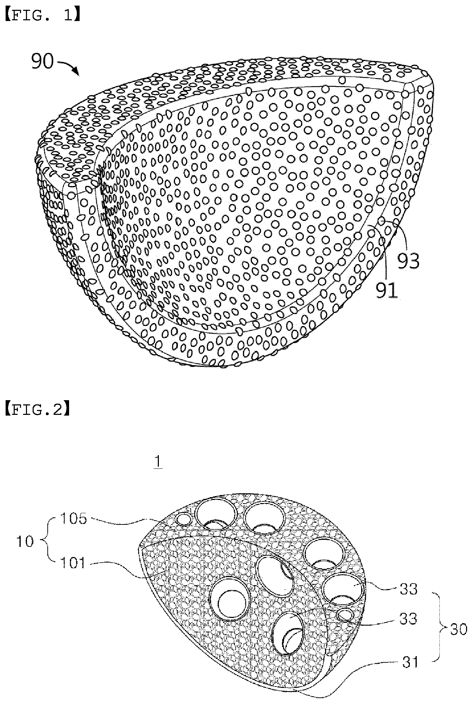

FIG. 1 is a view of a conventional porous augment implant 90, which is disclosed in Korean Patent Application Publication No. 10-2014-0031828 (published on March 13, 2014) .

The conventional porous augment implant 90 includes a first porous surface 91 in contact with a patient's orthopedic joint, and the first porous surface 91 has a plurality of pores sized to enable bone ingrowth in the augment implant. The augment implant further includes a plurality of protrusions 93 which is connected to the first porous surface 91 and protrudes as tall as 200**µm** to 400**µm** on the first porous surface 91. The plurality of protrusions 93 is configured to create friction with a bone surface without extending into a bone surface and without interfering with bone ingrowth into the plurality of pores.

According to the conventional technology 90, a patient's autogenous bone growth can be promoted due to the first porous surface 91 having multiple pores, so the augment implant and the patient's bone can be strongly combined with each other.

However, as in the conventional technology 90, when the augment implant is formed with only a porous material having multiple pores, a patient's autogenous bone growth can be promoted, but due to many pores formed in the augment implant, the mechanical strength of the augment implant is decreased.

That is, the porous augment implant having a relatively low strength may be easily damaged, such as being broken, cracked, or crushed even by a small external force.

In a process in which a porous part is damaged, porous particles, which are fragments of the porous part, may be generated. When these porous particles are introduced into the pores of the porous part, the porous particles may not be completely removed even after washing.

When the augment implant from which fine particles are not removed is implanted into a patient's body, the fine particles may come off from the implanted porous augment implant, and infiltrate the patient's blood vessels, and organs, etc., which may cause problems such as various inflammatory reactions and tissue necrosis.

Furthermore, the augment implant is often combined with an implant to be implanted together, and when a screw used in the process of combining the augment implant with the implant presses the porous augment implant, the porous augment implant having low strength is easily damaged by the screw, and due to this, a strong fixing force between the augment implant and the implant cannot be sufficiently secured.

In some cases, bone cement was used to solve this problem, but in this case, the problem of leakage of the bone cement leakage occurred. It has been reported that due to this, there occurred osteolysis, a phenomenon of a bone melting at a portion at which the implant and the patient's bone are in contact with each other. Furthermore, when bone cement is used, a large part of pores of a porous part was clogged. Accordingly, the porous part made to promote a patient's natural bone growth was clogged by the bone cement and failed to promote bone ingrowth.

Lastly, the conventional porous augment implant was manufactured by an additive manufacturing method called 3D printing. When there is empty space in an output printed by a 3D printer, a support which is a means which supports the output is required. In the absence of the support, a 3D printer laminates materials in the air. In order to print an output having a desired shape, an appropriate arrangement of the support is required to be designed.

However, as illustrated in FIG. 1, when the augment implant is formed as a structure having pores formed in the entirety of the augment implant, the support is unavoidably attached to a porous part having relatively low strength, and thus when removing the support from the porous augment implant after printing the support, even the porous part which is required to be prevented from being removed, together with the support, is removed, so the defective rate of finished products was high. Accordingly, a 3D printing manufacturing process is required to be performed again, thereby resulting in unnecessary waste of time, cost, and effort.

Accordingly, a related industry requires the introduction of a new technology in which a porous structure that maximizes a patient's bone growth is provided, a porous part is protected, and the removal of a support required during manufacturing by 3D printing is facilitated.

(Patent Document 1) Korean Patent Application Publication No. 10-2014-0031828 (published on March 13, 2014)

### Disclosure

### Technical Problem

The present disclosure has been made to solve above problems, and
an objective of the present disclosure is to compensate for damaged or missing bone parts in artificial joint replacement and to provide stability so that a patient's pain is alleviated and recovery of the patient is promoted.

Another objective of the present disclosure is to form a body part of a porous structure having multiple pores so as to induce high stability by surface roughness and autogenous bone ingrowth.

Still another objective of the present disclosure is to combine the body part of the porous structure with a frame part of a non-porous structure having strength higher than the porous structure so that mechanical strength of the body part is increased and damage to the body part due to external forces is prevented.

Still another objective of the present disclosure is to minimize the area of the frame part and to maximize the area of the body part so that bone growth is maximized.

Still another objective of the present disclosure is to ensure that the frame part does not close any surface of the body part even if the frame part of the non-porous structure is combined with the body part of the porous structure so that an augment implant can be inserted in various directions, not just in a specific direction.

Still another objective of the present disclosure is to form the frame part of a non-porous structure so that the body part of a porous structure having a relative low strength is protected and the decrease of porosity of the body part is minimized.

Still another objective of the present disclosure is to prevent any one surface of the body part from being blocked by the frame part even if the frame part is formed on the body par, thereby preventing the problem that when the frame part closes one surface of the body part, bone ingrowth may not be induced in the associated surface, and because of this, the associated surface may not be a bone contact surface which is required to have strong fixing force.

Still another objective of the present disclosure is to form a linear frame part on an edge which is a boundary between a surface and a surface in the body part so that the body part is protected to increase strength thereof, and to allow the body part to have high porosity in various directions so that bone growth can be promoted in any direction.

Still another objective of the present disclosure is to form an edge frame part along a contact edge which is a boundary between an implant contact surface of the body part which is a surface in contact with an implant and the bone contact surface of the body part which is a surface in contact with a bone so that an end part of the body part having low strength is prevented from being damaged, and to connect a support to the edge frame part when the augment implant is manufactured by 3D printing so that in a process of removing the support, the support is easily removed without damage to the porous structure.

Still another objective of the present disclosure is to allow the contact edge formed by the implant contact surface and the bone contact surface to be formed as a sharp edge so that when the augment implant is in contact with an implant, generation of empty space is minimized.

Still another objective of the present disclosure is to form a through hole to include a first tapered hole which has a truncated cone-shaped space by having a diameter decreasing gradually toward a second side from a first side; a second tapered hole which faces the first tapered hole and has a truncated cone-shaped space by having a diameter decreasing gradually toward the first side from the second side; and a connection hole which allows the first tapered hole and the second tapered hole to communicate with each other so that when inserting a fixing means such as a screw for fixing the augment implant into the through hole, the fixing means is inserted at various angles along a tapered inclined surface of the through hole, thereby enabling the augment implant to be fixed in various directions suitable for a patient's condition.

Still another objective of the present disclosure is to form a hole frame part inside the through hole formed in the body part so that when inserting a fixing means into the through hole for fixing the augment implant, the body part around the through hole is prevented from being damaged by an external force.

Still another objective of the present disclosure is to form the hole frame part in a tubular shape having open first and second surfaces such that the outer surface of the hole frame part has a shape complementary to the shape of the inner surface of the through hole, so that the hole frame part is stably fixed in the through hole to protect the through hole and is securely combined with the fixing means.

Still another objective of the present disclosure is to integrally manufacture the augment implant, in which the frame part of a non-porous structure is combined with the body part of a porous structure, by 3D printing.

Still another objective of the present disclosure is to accurately and easily manufacture a porous structure having micro-sized pores by using 3D printing.

### Technical Solution

In order to accomplish the above objectives, an augment implant of the present disclosure having the following components is realized according to embodiments.

According to one embodiment of the present disclosure, the augment implant of the present disclosure includes: a body part having a shape complementary to a shape of a bone; and a frame part which has a shape to prevent damage to the body part and is combined with the body part.

According to another embodiment of the present disclosure, the body part may be a porous structure having pores which induce bone ingrowth.

According to still another embodiment of the present disclosure, the frame part may be a non-porous structure having strength higher than the porous structure.

According to still another embodiment of the present disclosure, the frame part may not close any one surface of the body part so that the body part is able to be inserted in various directions.

According to still another embodiment of the present disclosure, the body part may include an edge which is a boundary between a surfaces and a surface of the body part.

According to still another embodiment of the present disclosure, the edge may include a contact edge which is a boundary between an implant contact surface of the body part which is a surface in contact with an implant and a bone contact surface of the body part which is a surface in contact with a bone.

According to still another embodiment of the present disclosure, the contact edge may be formed as a sharp edge which minimizes empty space between the implant and the implant contact surface.

According to still another embodiment of the present disclosure, the frame part may include an edge frame part formed on the contact edge.

According to still another embodiment of the present disclosure, the implant contact surface may have a shape complementary to a shape of an outer circumferential surface of the implant.

According to still another embodiment of the present disclosure, the body part may include a through hole formed through a second surface of the body part from a first surface of the body part, and the frame part may include a hole frame part formed inside the through hole.

According to still another embodiment of the present disclosure, the through hole may include a first tapered hole which has a truncated cone-shaped space by having a diameter decreasing gradually toward a second side from a first side, a second tapered hole which faces the first tapered hole and has a truncated cone-shaped space by having a diameter decreasing gradually toward the first side from the second side, and a connection hole which allows the first tapered hole and the second tapered hole to communicate with each other so that a multidirectional fixing of a fixing means is possible.

According to still another embodiment of the present disclosure, the hole frame part may be formed in a tubular shape having open first and second surfaces to cover an inner surface of the through hole, and an outer surface of the hole frame part may have a shape complementary to a shape of the inner surface of the through hole.

According to still another embodiment of the present disclosure, the augment implant may be integrally manufactured by 3D printing.

According to still another embodiment of the present disclosure, a support generated during 3D printing may be connected to the frame part.

### Advantageous Effects

The present disclosure can have the following effects by the above embodiments, components to described below, combination thereof, and use relationship thereof.

According to the present disclosure, it is possible to compensate for damaged or missing bone parts in artificial joint replacement and to provide stability, thereby alleviating a patient's pain and promoting recovery of the patient.

According to the present disclosure, the body part of a porous structure having multiple pores is formed, thereby inducing high stability by surface roughness and autogenous bone ingrowth.

According to the present disclosure, the body part of the porous structure is combined with the frame part of the non-porous structure having strength higher than the porous structure, thereby increasing mechanical strength of the body part and preventing damage to the body part due to external forces.

According to the present disclosure, the area of the frame part is minimized, and the area of the body part is maximized, thereby maximizing bone growth.

According to the present disclosure, even if the frame part of a non-porous structure is combined with the body part of a porous structure, the frame part does not close any one surface of the body part, thereby enabling the augment implant to be inserted in various directions, not just in a specific direction.

According to the present disclosure, the frame part of a non-porous structure is formed, thereby protecting the body part of a porous structure having relatively low strength and minimizing the decrease of porosity of the body part.

According to the present disclosure, even if the frame part is formed on the body part, any one surface of the body part is not closed by the frame part, thereby preventing the problem that when the frame part closes one surface of the body part, bone ingrowth may not be induced in the associated surface and because of this, the associated surface may not be the bone contact surface which is required to have strong fixing force.

According to the present disclosure, a linear frame part is formed on an edge which is a boundary between a surface and a surface in the body part, thereby protecting the body part and increasing strength thereof, and the body part has high porosity in various directions so that bone growth can be promoted in any direction.

According to the present disclosure, the edge frame part is formed along the contact edge which is a boundary between an implant contact surface of the body part which is a surface in contact with an implant and the bone contact surface of the body part which is a surface in contact with a bone, thereby preventing damage to an end part of the body part having low strength, and when the augment implant is manufactured by 3D printing, the support is connected to the edge frame part, thereby facilitating the removal of the support without damage to the porous structure in the process of removing the support.

According to the present disclosure, the contact edge formed by the implant contact surface and the bone contact surface is formed as the sharp edge, thereby minimizing the generation of empty space between the augment implant and an implant when bringing the augment implant into contact with the implant.

According to the present disclosure, the through hole includes the first tapered hole which has a truncated cone-shaped space by having a diameter decreasing gradually toward a second side from a first side, the second tapered hole which faces the first tapered hole and has a truncated cone-shaped space by having a diameter decreasing gradually toward the first side from the second side, and the connection hole which allows the first tapered hole and the second tapered hole to communicate with each other so that when inserting a fixing means such as a screw for fixing the augment implant into the through hole, the fixing means is inserted at various angles along the tapered inclined surface of the through hole, thereby enabling the fixing of the augment implant in various directions suitable for a patient's condition.

According to the present disclosure, the hole frame part is formed inside the through hole formed in the body part, thereby preventing the body part around the through hole from being damaged by an external force when inserting the fixing means into the through hole for fixing the augment implant.

According to the present disclosure, the hole frame part is formed in a tubular shape having open first and second surfaces such that the outer surface of the hole frame part has a shape complementary to the shape of the inner surface of the through hole so that the hole frame part is stably fixed in the through hole, thereby protecting the through hole and securely combining the hole frame part with the fixing means.

According to the present disclosure, the augment implant in which the frame part of a non-porous structure is combined with the body part of a porous structure can be integrally manufactured by 3D printing.

According to the present disclosure, a porous structure having micro-sized pores can be accurately and easily manufactured by using 3D printing.

### Description of Drawings

FIG. 1 is a view illustrating a conventional porous implant.
FIG. 2 is a view illustrating an augment implant according to an embodiment of the present disclosure viewed from an aspect.
FIG. 3 is a view illustrating the augment implant according to the embodiment of the present disclosure viewed from another aspect.
FIG. 4 is a view illustrating a body part of the present disclosure.
FIG. 5 is a view illustrating the combination of a body part having a round edge with an implant and the combination of a body part having a sharp edge with an implant.
FIG. 6 is a view illustrating a through hole of the present disclosure.
FIG. 7 is a view illustrating a frame part of the present disclosure.
FIG. 8 is a view illustrating a state in which a support is combined with the frame part.
FIG. 9 is a view illustrating a state in which the augment implant of the present disclosure is used.
FIG. 10 is a view illustrating a state in which the augment implant of the present disclosure is used.

### Best Mode

Hereinafter, exemplary embodiments of an augment implant according to the present disclosure will be described in detail with reference to the accompanying drawings. In the following description of the present disclosure, when it is determined that a detailed description of a known function or configuration may unnecessarily obscure the gist of the present disclosure, detailed description thereof will be omitted. Unless there is a special definition, all terms in this specification are the same as the general meaning of terms understood by those skilled in the art to which the present disclosure belongs, and when conflicting with the meaning of terms used in this specification, the terms used generally in the art follow the definition of the terms used herein.

The augment implant 1 of the present disclosure is intended to compensate for damaged or missing bone parts in artificial joint replacement, and is used to provide stability, alleviate a patient's pain, and promote recovery of the patient. The augment implant 1 is formed to have a porous structure to promote a patient's spontaneous bone ingrowth, so even if the augment implant 1 is implanted into a patient's body, the augment implant 1 may not cause any adverse reactions in the body and be properly combined with the patient's original bone. However, when the augment implant 1 has only the porous structure having multiple pores, the augment implant has decreased mechanical strength and may be easily crushed or broken due to an external force, and in this process, porous particles, which are fine particles, may be generated. Accordingly, the augment implant 1 has a porous structure as a whole and additionally has a separate structure in a specific part for reinforcing strength of the augment implant.

The manufacturing method of the augment implant 1 is not limited to any specific method, but may be preferably manufactured by 3D printing, which is an additive manufacturing method. As will be described later, the augment implant 1 is largely divided into a body part 10 of a porous structure and a frame part 30 of a non-porous structure. After the body part 10 is formed, the frame part 30 may be combined with the body part 10, or after the frame part 30 is formed, the body part 10 may be formed on the frame part 30. However, the body part 10 and the frame part 30 may be integrally manufactured by 3D printing. Accordingly, the augment implant 1 including a porous structure having micro- and nano-sized pores can be manufactured more accurately and easily.

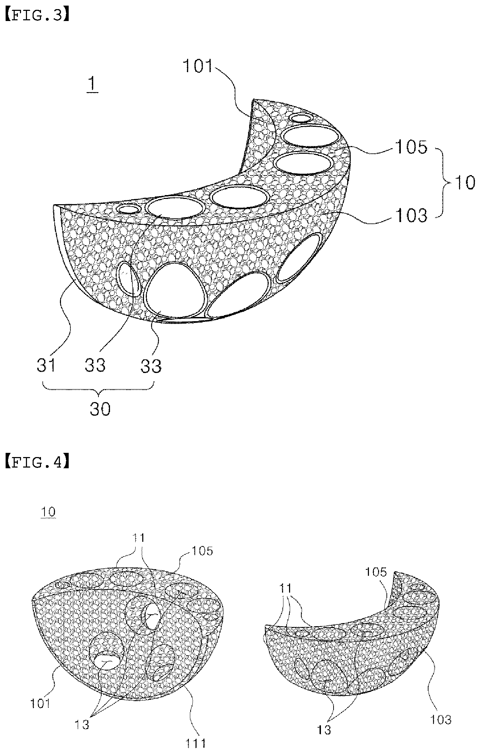

FIG. 2 is a view illustrating the augment implant according to the embodiment of the present disclosure viewed from an aspect, and FIG. 3 is a view illustrating the augment implant according to the embodiment of the present disclosure viewed from another aspect. Referring to FIGS. 2 and 3, the augment implant 1 of the present disclosure includes the body part 10 and the frame part 30.

The body part 10 is a component having a shape complementary to the shape of an area of a damaged bone and as illustrated in the drawings, is configured as a porous structure having pores which induce bone ingrowth. The body part 10 of a porous structure having multiple pores is formed to increase surface roughness of the body part 10, so the body part 10 can be stably seated on a bone, and bone ingrowth can be promoted through the multiple pores.

The body part 10 is not limited to any specific shape, and may be configured to have a shape suitable for filling a portion of a bone in which bone damage or defect has occurred. To this end, the body part 10 may be customized for a patient through the process of measuring and modeling a bone part of a patient requiring the augment implant 1 by reconstructing medical images such as MRI or CT, and through a 3D printing process thereof. The body part 10 has a surface, and the surface may be divided into an implant contact surface 101 which is a surface in contact with the implant prosthesis, a bone contact surface 103 which is a surface in contact with a bone, and a connecting surface 105 which connects the implant contact surface 101 with the bone contact surface 103. The augment implant 1 of the present disclosure may be preferably used for hip joint revision surgery, and is combined with the side of an acetabular cup, which is an implant prosthesis inserted into a patient's acetabular, to rebuild a damaged bone. To this end, the implant contact surface 101 of the body part 10 may be configured to have a shape complementary to an outer circumferential surface of the implant prosthesis.

FIG. 4 is a view illustrating the body part 10 of the present disclosure, and the body part 10 includes an edge 11 and a through hole 13.

The edge 11 means a boundary at which different surfaces of the body part 10 meet each other, and may be considered to mean a cornered edge of an object. That is, the edge 11 may be formed in a boundary between the implant contact surface 101 and the bone contact surface 103, a boundary between the bone contact surface 103 and the connecting surface 105, and a boundary between the connecting surface 105 and the implant contact surface 101.

The edge 11 includes a contact edge 111, and the contact edge 111 means a boundary between the implant contact surface 101 of the body part 10 which is a surface in contact with an implant and the bone contact surface 103 of the body part 10 which is a surface in contact with a bone. Preferably, the contact edge 111 may be formed as a sharp edge which minimizes empty space between the implant A and the implant contact surface 101.

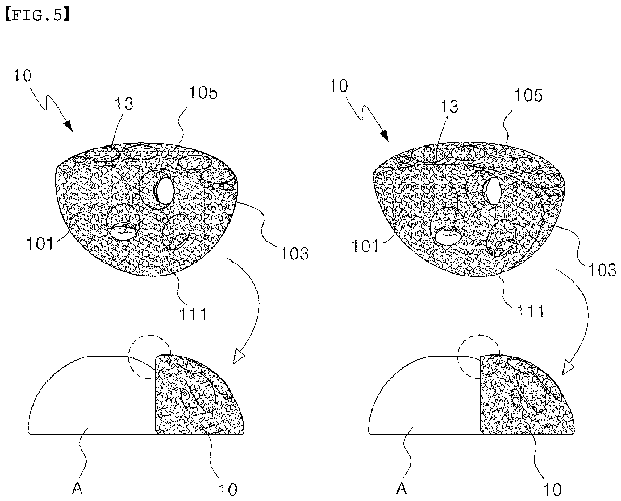

The sharp edge has concept opposite to a round edge, and FIG. 5 illustrates the combination of a body part 10 having a round edge with the implant A and the combination of a body part 10 having a sharp edge with the implant A. Referring to FIG. 5, it can be seen that in a case in which the contact edge 111 is formed to have a round edge, when the implant A and the augment implant 1 are combined with each other, a lot of empty space therebetween is generated. To solve this problem, in the augment implant of the present disclosure, the contact edge 111 is formed to have a sharp edge as illustrated at the right side of FIG. 5, and thus when the augment implant 1 is in contact with the implant A, the generation of empty space therebetween is minimized.

The through hole 13 refers to a hole formed through a second surface of the body part 10 from a first surface of the body part 10, and provides space into which a fixing means can be inserted. As illustrated in FIG. 4, the through hole 13 may be formed through the bone contact surface 103 from the implant contact surface 101 of the body part 10, and may be formed through the bone contact surface 103 from the connecting surface 105 of the body part 10. The through hole 13 may be preferably formed to have a circular cross-sectional shape so that a fixing means is easily inserted into the through hole 13. In addition, to secure the fixing force of the augment implant 1, the through hole 13 may include a plurality of through holes formed in the body part 10.

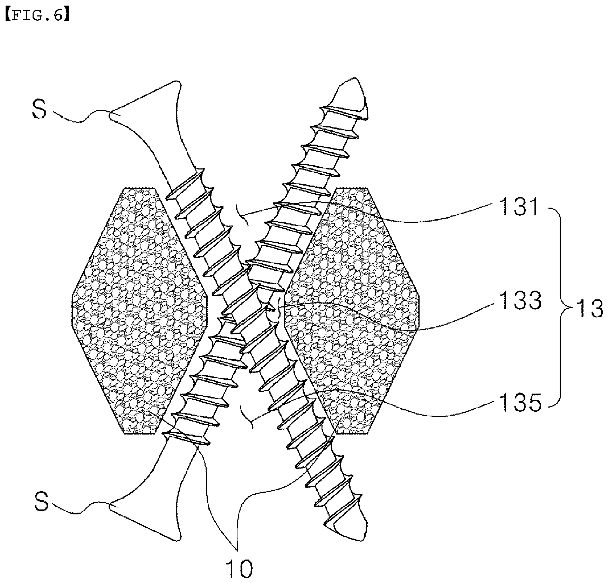

FIG. 6 is a view illustrating the through hole 13 of the present disclosure. Referring to FIG. 6, the through hole 13 includes a first tapered hole 131, a second tapered hole 133, and a connection hole 135.

The first tapered hole 131 refers to a component which has a truncated cone-shaped space by having a diameter decreasing gradually toward the second side from the first side. An inclined surface is formed by the first tapered hole 131, and a fixing means S guided along the inclined surface, such as a screw or bone cement, may be inserted at various angles and be fixed.

The second tapered hole 133 refers to a component which faces the first tapered hole 131 and has a truncated cone-shaped space by having a diameter decreasing gradually toward the first side from the second side. An inclined surface is formed even at a side opposite to the first tapered hole 131 by the second tapered hole 133, and thus in addition to the multidirectional fixing of the fixing means S toward the second side from the first side, the multidirectional fixing of the fixing means S toward the first side from the second side can be performed.

The connection hole 135 is configured to communicate the first tapered hole 131 and the second tapered hole 133 with each other and is not limited to any specific shape, but may preferably be considered as a component having a cylindrical space. The fixing means S may be inserted in a vertical direction through the connection hole 135.

The frame part 30, which has a shape to prevent damage to the body part 10 and is combined with the body part 10, is formed as the non-porous structure having higher strength than the porous structure. The non-porous structure, which has a concept opposite to the porous structure, may include a structure having no pore, and may include a structure of lower porosity than the porous structure in that the frame part 30, which reinforces the strength of the body part 10 and protects the porous structure, may have strength increasing as porosity decreases. The frame part 30 of the non-porous structure is provided to protect the body part 10 of a porous structure having a relatively low strength and increase the mechanical strength of the body part 10, thereby preventing damage to the body part 10 due to external forces and minimizing decrease of porosity of the body part 10.

More preferably, the frame part 30 does not close any one surface of the body part 10 so as to enable the multidirectional insertion of the body part 10. That is, even if the frame part 30 of a non-porous structure is combined with the body part 10 of a porous structure, the frame part 30 does not close any one surface of the body part 10, so the augment implant 1 may be inserted in various directions, not just in a specific direction. When the frame part 30 closes one surface of the body part 10, bone ingrowth may not be induced in the associated one surface, and because of this, the associated surface may not be the bone contact surface which is required to have strong fixing force. However, even if the frame part 30 is formed on the body part 10, any one surface of the body part 10 is prevented from being blocked by the frame part 30. Accordingly, since penetration of a bone into the body part can be performed in all directions, the augment implant 1 and a patient's bone can be strongly combined with each other after surgery.

As described above, in order to easily manufacture the augment implant 1 including a porous structure having a complicated shape, the augment implant 1 is preferably manufactured integrally by 3D printing. However, a method of stacking materials is used in a printing process by 3D printing, and thus when there is empty space in an output printed by a 3D printer, a means for supporting the output is required. Without the support means, the 3D printer may eject a material into the air, and the printed output may sag. The augment implant 1 of the present disclosure has a shape complementary to the anatomical shape of a patient, and in order to realize an atypical augment implant 1 in a pre-planned shape by 3D printing, it is necessary to print a support S supporting the atypical augment implant 1 together with the atypical augment implant 1.

The support S is finally removed from a completed augment implant 1. When the support S is combined with the body part 10 having a porous structure, in a process of removing the support S, the body part 10 having a relatively low strength may be removed together with the support S. In this case, the augment implant 1 is required to be reprinted and the waste of costs and time occurs.

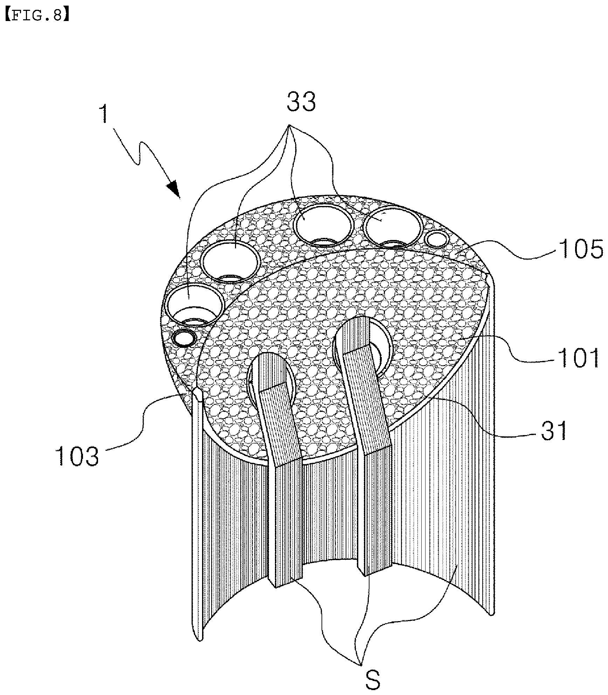

Accordingly, the augment implant 1 of the present disclosure is manufactured integrally by 3D printing, and the support S generated during 3D printing is connected to the frame part 30 as illustrated in FIG. 8. Accordingly, even if the support S is printed while being attached to the augment implant 1, the support S can be easily removed from the frame part 30 having relatively high strength and a smooth surface.

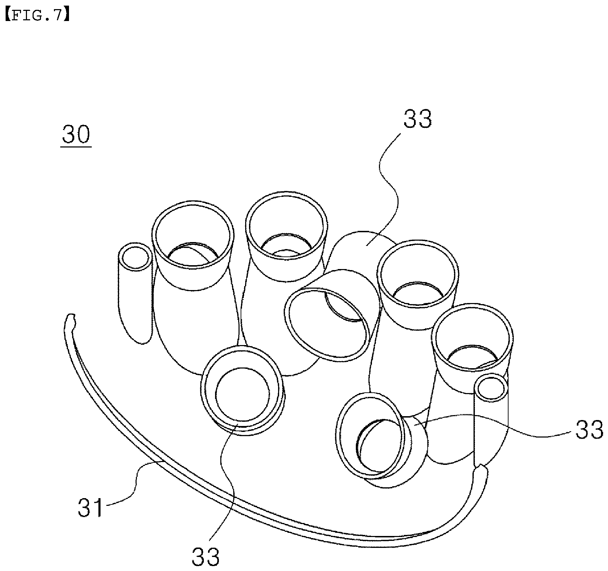

FIG. 7 is a view illustrating the frame part 30 of the present disclosure, and the frame part 30 includes an edge frame part 31 and a hole frame part 33.

The edge frame part 31 refers to the frame part 30 of the non-porous structure formed on the contact edge 111. The edge frame part 31 is formed along the contact edge 111 which is a boundary between the implant contact surface 101 of the body part 10 which is a surface in contact with an implant and the bone contact surface 103 of the body part 10 which is a surface in contact with a bone so that the end part of the body part 10 having low strength is prevented from being damaged, and when the augment implant 1 is manufactured by 3D printing, the support S is connected to the edge frame part 31 so that in the process of removing the support, the support is easily removed without damage to the porous structure. Above all, he linear edge frame part 31 is formed on an edge which is a boundary between a surface and a surface in the body part 10 to minimize the area of the frame part 30 and maximizing the area of the body part 10 so that bone growth can be maximized, and the body part 10 has high porosity in various directions so that bone growth can be promoted in any direction.

The hole frame part 33 is a frame part 30 formed inside the through hole 13 and is formed in a tubular shape having open first and second surfaces as illustrated in FIG. 7 so as to cover the inner surface of the through hole 13. When the fixing means is inserted into the through hole 13 to fix the augment implant 1 through the hole frame part 33, the body part 10 around the through hole 13 can be prevented from being damaged by external forces. It is preferable that the outer surface of the hole frame part 33 has a shape complementary to the shape of the inner surface of the through hole 13. Through this, the hole frame part 33 can be stably fixed in the through hole 13 to protect the through hole 13 and can be securely coupled to the fixing means. The hole frame part 33 may preferably be formed as many as the number of the through holes 13.

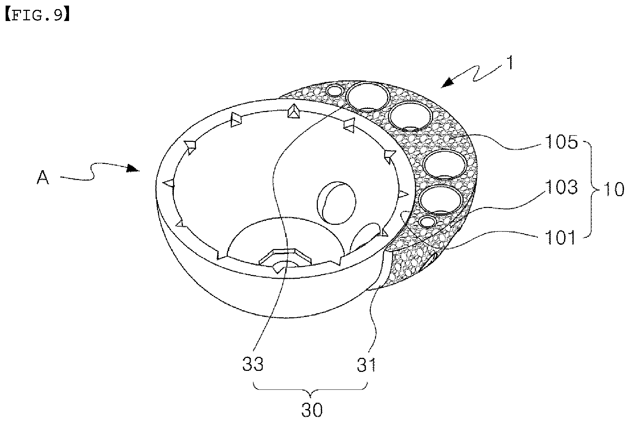

FIG. 9 is a view illustrating a state in which the augment implant of the present disclosure is used. Referring to FIG. 9, the augment implant 1 of the present disclosure may be used for hip joint revision surgery, etc., and may be combined with the side of an acetabular cup, which is an implant prosthesis inserted into a patient's acetabular, to rebuild a damaged bone. The augment implant 1 of the present disclosure is combined with the implant prosthesis A to reinforce the defective portion of a patient's bone. The augment implant 1 includes: the body part 10 of a porous structure which promotes bone ingrowth; the edge frame part 31 is formed along the contact edge 111 of the body part 10 to protect the body part 10 and increase strength thereof; and the hole frame part 33 formed inside the through hole 13 of the body part 10. In this case, any one surface of the body part 10 is not closed by the frame part 30, and thus the body part 10 can be inserted in various directions, and bone growth is possible in all directions. Furthermore, the edge 11 of the body part 10 includes the sharp edge, and thus when an implant A and the augment implant 1 are combined with each other, unnecessary space can be prevented from being defined therebetween. During 3D printing, the support is designed to be combined with the frame part 30, and thus in the process of removing the support, the body part 10 having a relative low strength may be prevented from being broken or removed together with the support.

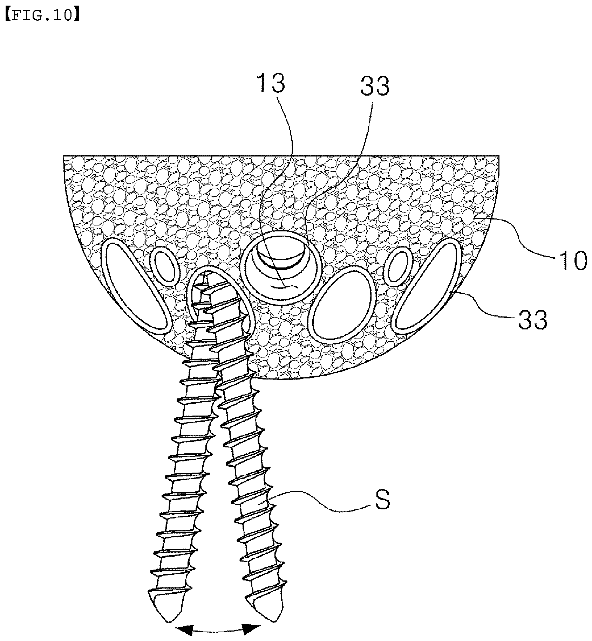

In addition, the through hole 13 includes: the first tapered hole 131 which has a truncated cone-shaped space by having a diameter decreasing gradually toward the second side from the first side; the second tapered hole 133 which faces the first tapered hole 131 and has a truncated cone-shaped space by having a diameter decreasing gradually toward the first side from the second side; and the connection hole 135 which allows the first tapered hole 131 and the second tapered hole 133 to communicate with each other. The augment implant has the hole frame part 33 which is formed in a tubular shape having open first and second surfaces and covers the inner surface of the through hole 13, and the outer surface of the hole frame part 33 has a shape complementary to the shape of the inner surface of the through hole 13. Accordingly, as illustrated in FIG. 10, the fixing means S such as a screw may be inserted at various angles along the inclined inner wall surface of the through hole 13, and in this process, due to the hole frame part 33, the through hole 13 having relatively low strength may not be damaged by external forces.

The above detailed description is to illustrate the present disclosure. In addition, the foregoing is intended to illustrate the exemplary embodiments of the present disclosure, and the present disclosure may be used in various combinations, variations, and environments. That is, changes or modifications are possible within the scope of the inventive concept disclosed in this specification, within a scope equivalent to the present disclosure and/or within the scope of skill or knowledge in the art. The embodiment of the present disclosure describes a best state for realizing the technical idea of the present disclosure, and various changes required in the specific application field and purpose of the present disclosure are also possible. Accordingly, the above detailed description of the present disclosure is not intended to limit the present disclosure to the disclosed embodiments. In addition, the appended claims should be construed to cover other embodiments as well.

## Claims

1. An augment implant comprising:
a body part having a shape complementary to a shape of a bone; and
a frame part which has a shape to prevent damage to the body part and is combined with the body part.

2. The augment implant of claim 1, wherein the body part is a porous structure having pores which induce bone ingrowth.

3. The augment implant of claim 2, wherein the frame part is a non-porous structure having strength higher than the porous structure.

4. The augment implant of claim 3, wherein the frame part does not close any one surface of the body part so that the body part is able to be inserted in various directions.

5. The augment implant of claim 1, wherein the body part comprises an edge which is a boundary between a surfaces and a surface of the body part.

6. The augment implant of claim 5, wherein the edge comprises a contact edge which is a boundary between an implant contact surface of the body part which is a surface in contact with an implant and a bone contact surface of the body part which is a surface in contact with a bone.

7. The augment implant of claim 6, wherein the contact edge is formed as a sharp edge which minimizes empty space between the implant and the implant contact surface.

8. The augment implant of claim 6, wherein the frame part comprises an edge frame part formed on the contact edge.

9. The augment implant of claim 6, wherein the implant contact surface has a shape complementary to a shape of an outer circumferential surface of the implant.

10. The augment implant of claim 1, wherein the body part comprises a through hole formed through a second surface of the body part from a first surface of the body part, and
the frame part comprises a hole frame part formed inside the through hole.

11. The augment implant of claim 10, wherein the through hole comprises a first tapered hole which has a truncated cone-shaped space by having a diameter decreasing gradually toward a second side from a first side, a second tapered hole which faces the first tapered hole and has a truncated cone-shaped space by having a diameter decreasing gradually toward the first side from the second side, and a connection hole which allows the first tapered hole and the second tapered hole to communicate with each other so that a multidirectional fixing of a fixing means is possible.

12. The augment implant of claim 11, wherein the hole frame part is formed in a tubular shape having open first and second surfaces to cover an inner surface of the through hole, and an outer surface of the hole frame part has a shape complementary to a shape of the inner surface of the through hole.

13. The augment implant of any one of claims 1 to 12, wherein the augment implant is integrally manufactured by 3D printing.

14. The augment implant of claim 13, wherein a support generated during 3D printing is connected to the frame part.
